# EUROPÄISCHE PATENTSCHRIFT

(11) **EP 0 880 493 B1**
(45) Veröffentlichungstag und Bekanntmachung des Hinweises auf die Patenterteilung: **17.05.2000**
(21) Anmeldenummer: 97904407.0
(22) Anmeldetag: 10.02.1997
(51) Int. Cl.: C07C 45/38, C07C 47/04, B01J 27/18, B01J 37/34

(54) **VERFAHREN ZUR HERSTELLUNG VON FORMALDEHYD**
PROCESS FOR THE PREPARATION OF FORMALDEHYDE
PROCEDE DE FABRICATION DU FORMALDEHYDE

(30) Priorität: 13.02.1996 DE 19605211
(43) Veröffentlichungstag der Anmeldung: 02.12.1998
(73) Patentinhaber: BASF AKTIENGESELLSCHAFT, 67056 Ludwigshafen (DE)
(72) Erfinder: DIERCKS, Rainer, D-67141 Neuhofen (DE); KNUTH, Bernhard, D-67246 Dirmstein (DE)
(86) Internationale Anmeldenummer: EP9700600
(87) Internationale Veröffentlichungsnummer: WO9730013

(56) Entgegenhaltungen:
- EP-A- 0 467 169
- EP-A- 0 624 565
- US-A- 4 233 248
- CHEMICAL ABSTRACTS, vol. 105, no. 24, 15.Dezember 1986 Columbus, Ohio, US; abstract no. 210789, DENG J ET AL: "Silver-phosphorus catalyst and its application in making aldehyde by catalytic oxidation of alcohol" XP002028603 & CN 85 100 530 A (FUDAN UNIVERSITY;PEOP. REP. CHINA) 10.März 1986

## Beschreibung

Die vorliegende Erfindung betrifft ein Verfahren zur Herstellung von Formaldehyd durch oxidative Dehydrierung von Methanol.

Verfahren zur Herstellung von Formaldehyd durch oxidative Dehydrierung von Methanol an einem aus Silberkristallen aufgebauten Katalysator-Festbett sind allgemein bekannt (vgl. Ullmanns Enzyklopädie der technischen Chemie, 3. Auflage, Urban und Schwarzenberg, München-Berlin, 1956, 7. Band, S. 660 bis 663). Nach diesem Verfahren wird Silber in einer elektrolytischen Zelle anodisch zu Silberionen oxidiert und kathodisch wieder zu Silber reduziert. Das an der Kathode gebildete grobkristalline Silber eignet sich als Katalysator zur Formaldehydsynthese aus Methanol.

Vorteilhafte Effekte, die bei der Verwendung von Phosphorverbindungen als Promotoren zur Oxidation von Methanol zu Formaldehyd in Gegenwart eines Silberkatalysators auftreten, sind weiterhin aus der CN-A-85100530, DE-A-4022603 und der JP-A-38227/83 bekannt.

Die EP-A-0 467 169 beschreibt die Herstellung von Formaldehyd durch oxidative Dehydrierung von Methanol an einem Katalysator-Festbett, das aus Schichten von Silberkristallen aufgebaut ist, die ein pulverförmiges phosphorhaltiges Salz als Promotor enthalten, indem man eine Gasmischung, die Methanol und Sauerstoff enthält, durch das Katalysator-Festbett leitet. Die Mitverwendung von Distickstoffoxid in der Gasmischung wird nicht empfohlen.

Aus der EP-A-0624565 ist die Herstellung von Formaldehyd durch oxidative Dehydrierung von Methanol an einem Silberkatalysator-Festbett bekannt, indem man eine Gasmischung enthaltend Methanol, Sauerstoff und Distickstoffoxid durch den Katalysator leitet. Es findet sich kein Hinweis auf die Verwendung eines phosphordotierten Silberkatalysator-Festbetts.

Die vorstehend genannten Verfahren zur Herstellung von Formaldehyd ermöglichen zwar die Herstellung von Formaldehyd aus Methanol mit beachtlicher Wirtschaftlichkeit. Im Hinblick darauf, daß dieses Verfahren als industrielles Großverfahren genutzt wird, erscheint die damit erzielbare Ausbeute noch verbesserungsfähig.

Die Aufgabe der vorliegenden Erfindung bestand deshalb darin, ein Verfahren zu entwickeln, das eine weitere Verbesserung der Wirtschaftlichkeit der Formaldehydherstellung ermöglicht, insbesondere was die Ausbeute bei der oxidativen Dehydrierung des Methanols betrifft.

Demgemäß wurde ein Verfahren zur Herstellung von Formaldehyd durch oxidative Dehydrierung von Methanol gefunden, welches dadurch gekennzeichnet ist, daß man eine Gasmischung (i), enthaltend
a) 0,1 bis 50, bevorzugt 10 bis 40 Vol.-% Methanol,
b) 0,1 bis 30, bevorzugt 5 bis 20 Vol.-% Sauerstoff,
c) 0,1 bis 50, bevorzugt 1 bis 20 Vol.-% Distickstoffoxid und
d) 0 bis 60, bevorzugt 10 bis 50 Völ.-% Wasser
bei einer Temperatur von 150 bis 800°C durch ein phosphordotiertes Silberkatalysator-Festbett leitet.

Diese Angaben beziehen sich auf einen Druck von 1 bar.

Dabei beträgt das Mol-Verhältnis Methanol zu Distickstoffoxid günstigerweise 1 : 1 bis 30 : 1.

Für das erfindungsgemäße Verfahren eignen sich beispielsweise phosphordotierte Silberkatalysatoren, die erhältlich sind, indem man
Ia. Silberkristalle, die man durch elektrolytische Abscheidung von Silber aus einer wässerigen Silbersalzlösung erhält, zu einem Ausgangs-Silberkatalysator-Festbett (a) anordnet und
IIa.das Ausgangs-Silberkatalysator-Festbett mit 1 bis 20000 Gew.-ppm Phosphor, bezogen auf das Silber, in Form einer feinverteilten Phosphorverbindung mit einer Schmelz- bzw. Zersetzungstemperatur von mehr als 500°C (Phosphorverbindung P), in Kontakt bringt.

Die in Schritt Ia beschriebene Herstellung der Silberkristalle ist allgemein bekannt (vgl. Ullmann's Enzyklopädie der technischen Chemie, 3. Auflage, Urban und Schwarzenberg, München-Berlin, 1956, 7. Band, S. 660 bis 663). Besonders gute Ergebnisse lassen sich mit den in der DE-A-2322757 beschriebenen Ausgangs-Katalysator-Festbetten erreichen.

Geeignete Silberkristalle erhält man insbesondere dann, wenn man die Elektrolyse nach dem in der deutschen Patentschrift 1166171 beschriebenen Verfahren durchführt.

Bevorzugt wird als Elektrolyt eine wäßrige Silbernitratlösung eingesetzt. Diese Silbernitratlösung hat im allgemeinen einen pH-Wert von 1 bis 4 und enthält 1 bis 5 Gew.-% Silber. Der pH-Wert wird günstigerweise mit Salpetersäure eingestellt.

Als Elektroden dienen die üblicherweise bei der Elektrolyse von Silber verwendeten Elektroden. Geeignete Anoden sind Säcke, in die das zu oxidierende Silber im allgemeinen als Granulat oder als Pulver eingefüllt worden ist. Als Kathoden kommen insbesondere Silberbleche in Betracht.

Die Elektrolyse wird günstigerweise bei Stromdichten von 80 bis 500 A/m² Kathodenfläche und Elektrolyttemperaturen von 10 bis 30°C durchgeführt.

Um diese Stromdichten zu erreichen, sind bei den meisten Elektrolysezellen Spannungen von 1 bis 15 Volt erforderlich.

Es empfiehlt sich, die an der Kathode gebildeten Silberkristalle fortwährend von der Kathode zu entfernen. Es werden im allgemeinen Silberkristalle mit einer Korngröße von 0,2 bis 5 mm erhalten.

Meistens reicht eine einmalige Elektrolyse aus, um brauchbare Silberkristalle zu erhalten.

Im allgemeinen ordnet man die Silberkristalle zu einem Ausgangs-Silberkatalysator-Festbett an, das aus 1 bis 9 Schichten von Silberkristallen besteht und eine Gesamtschichtdicke von 1 bis 10 cm aufweist. Derartige Festbetten, die auch als "Kurzschichten" bezeichnet werden, sind allgemein bekannt (vgl. Ullmanns Enzyklopädie der technischen Chemie, 4. Auflage, Verlag Chemie -Weinheim-New York, Band 13, S. 539 bis 541).

Im Schritt IIa wird das Ausgangs-Silberkatalysator-Festbett Ia mit 1 bis 20000, bevorzugt 5 bis 5000 Gew.-ppm Phosphor, bezogen auf das Silber, in Form einer feinverteilten Phosphorverbindung mit einer Schmelz- bzw. Zersetzungstemperatur von mehr als 500°C (Phosphorverbindung P), in Kontakt gebracht.

Als Phosphorverbindungen (P) kommen phosphorhaltige Salze in Betracht. Beispiele hierfür sind die in der DE-A-4022603 genannten phosphorhaltigen Salze, z.B. anorganische Phosphate von Alkali-, Erdalkali-, und Schwermetallen wie Ag, Zn und Fe oder von Bor und Ammonium.

Bevorzugt sind Phosphate oder Pyrophosphate von Alkali- oder Erdalkalimetallen, z.B. Na₄P₂O₇, Li₃PO₄, Mg₃(PO₄)₂, Ca₃(PO₄)₂.

Im allgemeinen geht man dabei so vor, daß man ein feinteiliges Pulver der Phosphorverbindung (P) auf das aktivierte Silberkatalysator-Festbett aufstreut oder es mit einer Lösung der Phosphorverbindung (P) imprägniert und das Lösungsmittel verdunsten läßt. Die Korngröße der als Pulver eingesetzten Phosphorverbindung (P) ist nicht kritisch, im allgemeinen beträgt sie ca. 1 mm bis 1 µm.

Bei den Lösungen der Phosphorverbindungen (P) handelt es sich im allgemeinen um wässerige Lösungen, die 0,01 bis 50 Gew.-% der Phosphorverbindung (P) enthalten. Zur Imprägnierung des aktivierten Silberkatalysator-Festbetts wird es mit einer dieser Lösungen getränkt oder besonders vorteilhaft die Lösungen auf das aktivierte Silberkatalysator-Festbett aufgesprüht und das Lösungsmittel anschließend verdunstet.

Die Menge an aufgesprühter oder aufgestreuter Phosphorverbindung (P) wird bevorzugt so gewählt, daß die Menge an Phosphor 0,01 bis 100, bevorzugt 0,05 bis 10 mg pro cm² der phosphordotierten Silberkatalysator-Festbett-Querschnittsfläche beträgt.

Die auf diese Weise hergestellten Silberkatalysator-Festbetten (a) entfalten beim Beginn der Durchleitung des Gasgemisches (i) im allgemeinen nicht von Anfang an ihre volle katalytische Aktivität. Es hat sich deshalb als zweckmäßig erwiesen, die phosphordotierten Silberkatalysator-Festbetten (a) bei Beginn der Formaldehydherstellung zu aktivieren.

Die Aktivierung des Katalysators kann man beispielsweise vornehmen, indem man den Katalysator unmittelbar vor Beginn der Durchleitung des Gasgemisches (i) auf eine Temperatur von 300 bis 400°C vorheizt und/oder ein auf eine Temperatur von 100 bis 800, bevorzugt 200 bis 700°C vorgeheiztes Gasgemisch (i) oder eine Gasmischung, die Methanol und Sauerstoff, jedoch weniger als 0,1 Vol-%. Distickstoffoxid enthält (Gasmischung ii), durch das Festbett leitet. Die Gasmischung (ii) enthält im allgemeinen 0,25 bis 0,60, bevorzugt 0,35 bis 0,50 mol Sauerstoff pro Mol Methanol und 0,2 bis 3,0, bevorzugt 0,67 bis 1,75 mol Wasser pro Mol Methanol und 0,9 bis 2,3, bevorzugt 1,3 bis 1,8 mol Stickstoff pro Mol Methanol. Die Menge an Methanol in Form der Gasmischung (i) bzw. (ii), die man bei Beginn der Aktivierungsphase, die üblicherweise 0,1 bis 100 Stunden dauert, pro Stunde und pro cm² Querschnittsfläche des phosphordotierten Silberkatalysator-Festbetts (a) durch dieses leitet, beträgt 0,001 bis 1 kg. Die Menge an Gasmischung (i) bzw. (ii), die man pro Zeiteinheit durch das phosphordotierte Silberkatalysator-Festbett (a) leitet, wird während der Aktivierungsphase kontinuierlich auf den Wert der Endbelastung erhöht, der im allgemeinen 0,01 bis 5, bevorzugt 0,1 bis 5 kg beträgt. Im allgemeinen erübrigt sich das Vorheizen der Gasmischung (i) bzw. (ii) spätestens nach Ende der Aktivierungsphase, da sich das Festbett durch die freiwerdende Reaktionswärme auf die erforderliche Temperatur aufheizt.

Damit sich Aktivierungsphase und die Herstellung von Formaldehyd nach dem erfindungsgemäßen Verfahren nahtlos aneinander anschließen können, nimmt man die Aktivierung des Ausgangs-Katalysator-Festbetts (a) zweckmäßigerweise in einem Festbettreaktor vor, wie man ihn üblicherweise für die Herstellung von Formaldehyd durch oxidative Dehydrogenierung von Methanol verwendet und leitet durch diesen kontinuierlich die Gasmischung (i) bzw. (ii). Vorzugsweise steht der Reaktor dabei senkrecht und die Gasmischung (i) bzw. (ii) wird von oben nach unten durch den Reaktor geleitet. Solche Reaktoren bzw. Verfahren sind beispielsweise in der EP-A-467 169, der DE-A-2444586 und der EP-A-0150436 beschrieben.

Günstigerweise wählt man die Querschnittsfläche des Reaktors und des Ausgangs-Silberkatalysator-Festbetts (b) gleich und ordnet es so in dem Reaktor an, daß die Schichten der Silberkristalle senkrecht zur Strömungsrichtung der Gasmischung (i) bzw. (ii) liegen.

Ferner sind für das erfindungsgemäße Verfahren phosphordotierte Silberkatalysator-Festbetten einsetztbar, die erhältlich sind, indem man
Ib. Silberkristalle, die man durch elektrolytische Abscheidung von Silber aus einer wässerigen Silbersalzlösung erhält, zu einem Ausgangs-Silberkatalysator-Festbett (b) anordnet,
IIb. aus dem Ausgangs-Silberkatalysator-Festbett (b) ein aktiviertes Silberkatalysator-Festbett (b) herstellt, indem man durch dieses (das Ausgangs-Silberkatalysator-Festbett (b)) bei einer Temperatur von 150 bis 800°C eine Gasmischung, enthaltend Methanol und Sauerstoff, jedoch weniger als 0,1 Vol-% Distickstoffoxid (Gasmischung ii), oder die Gasmischung (i) leitet, und
IIIb. das aktivierte Silberkatalysator-Festbett (b) mit 1 bis 20000 Gew.-ppm, Phosphor, bezogen auf das Silber, in Form einer feinverteilten Phosphorverbindung (P) in Kontakt bringt.

Die Herstellung des Ausgangs-Silberkatalysator-Festbetts (b) in Schritt (Ib) nimmt man vorzugsweise auf gleiche Art und Weise vor wie die des Ausgangs-Silberkatalysator-Festbetts (a).

In Schritt (IIb) leitet man zur Aktivierung des Ausgangs-Silberkatalysator-Festbetts (b) bei einer Temperatur von 100 bis 800, bevorzugt 200 bis 700°C bevorzugt kontinuierlich eine Gasmischung (i) oder (ii) hindurch. Um die definitionsgemäße Temperatur zu erreichen, ist es zweckmäßig, bei Beginn der Durchleitung die Gasmischung (i) bzw. (ii) selbst auf diese Temperaturen vorzuheizen. Im allgemeinen erübrigt sich das Vorheizen der Gasmischung (i) bzw. (ii) nach einer gewissen Zeit, da sich das Festbett durch die freiwerdende Reaktionswärme auf die erforderliche Temperatur aufheizt.

Im allgemeinen reicht es zur Herstellung des aktivierten Silberkatalysator-Festbetts (b) aus, wenn man durch das Ausgangs-Silberkatalysator-Festbett (b) bei einer Temperatur von 100 bis 800, bevorzugt von 200 bis 700°C, pro cm² Querschnittsfläche des Ausgangs-Silberkatalysator-Festbetts 0,0001 bis 0,5, bevorzugt 0,01 bis 0,5 kg Methanol in Form der Gasmischung (i) bzw. (ii), durch dieses (das Ausgangs-Silberkatalysator-Festbett) leitet. Die Durchleitung der angegebenen Mengen der Gasmischungen (i) bzw. (ii) erfolgt günstigerweise mit einer Geschwindigkeit, die im allgemeinen so gewählt wird, daß die Durchleitung 0,01 bis 500, bevorzugt 0,1 bis 100, besonders bevorzugt 1 bis 50 h erfordert.

Ansonsten kann man die Durchführung von Schritt IIb auf die gleiche Weise vornehmen wie die Aktivierung des phosphordotierten Silberkatalysators (a).

Bereits während der Aktivierungsphase wird das Methanol, das in der Gasmischung (i) bzw. (ii) enthalten ist, nahezu quantitativ zu Formaldehyd umgesetzt. Das bedeutet, daß man Schritt IIb auch schon für die Produktion von Formaldehyd nutzen kann, auch wenn die Raum-Zeit-Ausbeute noch nicht ganz so hoch ist wie bei dem vollständig aktivierten phosphordotierten Silberkatalysator-Festbett.

Im Schritt IIIb wird das aktivierte Silberkatalysator-Festbett mit 1 bis 20000 Gew.-ppm Phosphor, bezogen auf das Silber, in Form einer feinverteilten Phosphorverbindung mit einer Schmelz- bzw. Zersetzungstemperatur von mehr als 500°C (Phosphorverbindung P), in Kontakt gebracht.

Bezüglich der bevorzugt angewandten Mengen an Phosphorverbindung (P), der bevorzugten Phosphorverbindungen (P) und ihrer angewandten Mengen sowie der Art und Weise des Aufbringens auf das aktivierte Silberkatalysator-Festbett gilt hier das gleiche wie bei Schritt IIa.

Bevorzugt nimmt man das Aufbringen der Phosphorverbindung (P) auf das aktivierte Silberkatalysator-Festbett (b) vor, ohne dabei die Durchleitung des Gasstromes (i) bzw. (ii) zu unterbrechen.

Diese Vorgehensweise hat den Vorteil, daß sich, sofern die Herstellung des phosphordotierten Silberkatalysator-Festbettes in einem Festbettreaktor vorgenommen wird, der auch für die oxidative Dehydrogenierung von Methanol zu Formaldehyd geeignet ist, die Herstellung von Formaldehyd aus der Gasmischung (i) unter Verwendung des erfindungsgemäßen phosphordotierten Silberkatalysator-Festbettes unmittelbar an dessen Herstellung anschließen kann. Auf diese Weise ist es möglich, die Herstellung des phosphordotierten Silberkatalysator-Festbetts, bei der auch schon Formaldehyd produziert werden kann und die Produktion von Formaldehyd unter Verwendung des erfindungsgemäßen Katalysator-Festbetts besonders rationell zu kombinieren.

Es ist weiterhin möglich, ein phosphordotiertes Silberkatalysator-Festbett mit maximaler Aktivität in einem iterativen Verfahren herzustellen, indem man in Schritt IIIb zunächst nur einen Teil, zweckmäßigerweise 0,01 bis 2 mg, bevorzugt 0,05 bis 1 mg Phosphor, pro cm² der Querschnittsfläche des aktivierten Silberkatalysator-Festbetts in Form der Phosphorverbindung (P) auf dieses aufbringt und die Menge an Phosphorverbindung (P) schrittweise, beispielsweise in Schritten von 1 bis 100 %, bezogen auf die anfangs aufgebrachte Menge, erhöht und gleichzeitig die Ausbeute an Formaldehyd kontrolliert. Dabei wird die aufgetragene Menge an Phosphorverbindung (P) schrittweise solange erhöht, bis keine Ausbeutesteigerung durch weiteres Aufbringen der Phosphorverbindung (P) mehr erreicht werden kann.

Die phosphordotierten Silberkatalysator-Festbetten verlieren bei Ihrer Verwendung im erfindungsgemäßen Verfahren langsam stetig an Aktivität, was an sinkender Ausbeute an Formaldehyd bemerkbar ist.

Dieser Aktivitätsverlust der erfindungsgemäßen Katalysatoren läßt sich teilweise vermeiden, wenn man pro kg Methanol in Form der Gasmischung (i), das man, bezogen auf 1 cm² der Querschnittsfläche des phosphordotierten Silberkatalysator-Festbetts, durch dieses leitet, zusätzlich entweder kontinuierlich oder diskontinuierlich (in jeweils einer Portion nach Einleitung einer definierten Menge an Gasmischung (i)) 0,01 bis 100 Gew.-ppm Phosphor, bezogen auf das phosphordotierte Silberkatalysator-Festbett, in Form der Phosphorverbindung (P) auf dieses aufbringt, bevorzugt ohne die Einleitung der Gasmischung (i) zu unterbrechen. Bei kontinuierlichem Aufbringen kann der Aktivitätsverlust verlangsamt, bei der schrittweisen diskontinuierlichen Aufbringung teilweise rückgängig gemacht werden.

Das nachträgliche Aufbringen der Phosphorverbindung (P) kann auf die gleiche Weise erfolgen, wie es in Schritt IIb bei der Herstellung des phosphordotierten Silberkatalysator-Festbetts beschrieben ist.

Wird das nachträgliche Aufbringen der Phosphorverbindung (P) diskontinuierlich vorgenommen, so werden die Zeitintervalle zwischen dem nachträglichen Aufbringen der Phosphorverbindung (P) auf das phosphordotierte Silberkatalysator-Festbett günstigerweise so lange gewählt, daß in dieser Zeit nicht mehr als 500, bevorzugt 1 bis 50 kg Methanol in Form der Gasmischung (M), bezogen auf 1 cm² der Querschnittsfläche des phosphordotierten Silberkatalysator-Festbetts, durch dieses geleitet werden, da ansonsten zwischenzeitlich die Ausbeute zu stark absinken würde.

Das Herstellungsverfahren von Formaldehyd durch oxidative Dehydrogenierung von Methanol unter Verwendung des erfindungsgemäßen Katalysator-Festbetts wird im übrigen in an sich bekannter Weise durchgeführt, indem man das Gasgemisch (i) bei Temperaturen von etwa 500 bis 750°C, insbesondere 600 bis 710°C durch das phosphordotierte Silberkatalysator-Festbett leitet. Das Verfahren wird im allgemeinen bei einem Druck von 0,5 bis 2 bar, vorzugsweise von 0,8 bis 1,8 bar kontinuierlich durchgeführt. Dabei ist es vorteilhaft, die die Katalysatorzone verlassenden Reaktionsgase innerhalb kurzer Zeit abzukühlen, z.B. auf Temperaturen von 50 bis 350°C. Das abgekühlte Gasgemisch wird dann zweckmäßigerweise einem Absorptionsturm zugeführt, in welchem der Formaldehyd mit Wasser oder einer wässerigen Formaldehyd-Harnstoff-Lösung aus dem Gasgemisch gewaschen wird.

Spezielle vorteilhafte Varianten des allgemein bekannten Verfahrens zur Herstellung von Formaldehyd, die auch beim erfindungsgemäßen Verfahren angewendet werden können, sind in der DE-A-2444586, der DE-A-2451990, der EP-A-0083427 und der EP-A-0150436, empfohlen.

Das erfindungsgemäße Verfahren zeichnet sich insbesondere dadurch aus, daß sich mit ihm Formaldehyd besonders wirtschaftlich herstellen läßt, weil bei ihm die Ausbeute besonders hoch ist.

### Beispiel 1

In einem senkrecht stehenden Versuchsreaktor mit einem Innendurchmesser von 15 cm wurde ein dreischichtiges Ausgangs-Silberkatalysator-Festbett gleichen Durchmessers mit einer Gesamtschichtdicke von 2 cm angebracht. Die untere Schicht bestand aus 1000 g Silberkristallen der Korngröße 1 bis 2,5 mm, die mittlere Schicht aus 65 g Silberkristallen der Korngröße von 0,75 bis 1 mm und die obere Schicht aus 185 g Silberkristallen der Korngröße 0,2 bis 0,75 mm.

Durch dieses Ausgangs-Silberkatalysator-Festbett, das auf 340°C aufgeheizt worden war, wurde eine Gasmischung (ii), bestehend aus Methanol, Wasser und Luft geleitet. Die Menge wurde während der 23stündigen Aktivierungsperiode auf pro Stunde 32 kg Methanol, 21,4 kg Wasser und 54 kg Luft erhöht (Endbelastung). Am Ende der Aktivierungsperiode betrug die Temperatur in dem Festbett 700°C. Dieser Mengenstrom wurde während des gesamten Versuchsdauer konstant gehalten. Anschließend wurden 0,6 mg Phosphor pro cm² aktiviertes Silberkatalysator-Festbett-Querschnitt in Form einer 3gew.-%igen wässerigen Lösung von Na₄P₂O₇ auf die Oberfläche des aktiviertes Silberkatalysator-Festbett aufgesprüht, wobei die Zufuhr der Gasmischung fortgesetzt wurde. Anschließend wurde die Gasmischung (ii) durch eine Gasmischung (i), bestehend aus 7,8 kg N₂O, 30 kg Methanol, 21,4 kg Wasser und 35 kg Luft ersetzt.

### Beispiel 2

Auf ein Ausgangs-Silberkatalysator-Festbett, das, wie in Beispiel 1 beschrieben, hergestellt worden war, wurden 1,2 g Na₄P₂O₇ aufgestreut. Der Katalysator wurde zunächst 42 h mit der in Beispiel 1 angegebenen Gasmischung (ii) aktiviert, wonach dieses durch die Gasmischung (i) ersetzt wurde.

### Vergleichsbeispiel 1

Es wurde wie bei Beispiel 2 beschrieben vorgegangen, jedoch wurde nach der Aktivierungsperiode die Zufuhr der Gasmischung (ii) nicht durch die der Gasmischung (i) ersetzt, sondern fortgesetzt.

### Vergleichsbeispiel 2

Es wurde wie bei Beispiel 2 beschrieben vorgegangen, jedoch wurde das Ausgangs-Silberkatalysator-Festbett nicht mit Phosphor dotiert.

Die Ergebnisse der durchgeführten Versuche sind der nachstehenden Tabelle zu entnehmen.

| | Umsatz [%] | Selektivität [%] | Ausbeute [%] |
|---|---|---|---|
| Bsp. 1 | 98,3 | 93,9 | 92,3 |
| Bsp. 2 | 97,7 | 93,1 | 91,0 |
| Vglbsp. 1 | 98,1 | 92,1 | 90,4 |
| Vglbsp. 2 | 98,9 | 91,4 | 90,4 |

## Patentansprüche

1. Verfahren zur Herstellung von Formaldehyd durch oxidative Dehydrierung von Methanol, dadurch gekennzeichnet, daß man eine Gasmischung (i), enthaltend
a) 0,1 bis 50 Vol.-% Methanol,
b) 0,1 bis 30 Vol.-% Sauerstoff,
c) 0,1 bis 50 Vol.-% Distickstoffoxid und
d) 0 bis 60 Vol.-% Wasser
bei einer Temperatur von 150 bis 800°C durch ein phosphordotiertes Silberkatalysator-Festbett leitet.

2. Verfahren nach Anspruch 1, dadurch gekennzeichnet, daß man ein phosphordotiertes Silberkatalysator-Festbett (a) einsetzt, welches erhältlich ist, indem man
Ia. Silberkristalle, die man durch elektrolytische Abscheidung von Silber aus einer wässerigen Silbersalzlösung erhält, zu einem Ausgangs-Silberkatalysator-Festbett (a) anordnet,
IIa. das Ausgangs-Silberkatalysator-Festbett mit 1 bis 20000 Gew.-ppm Phosphor, bezogen auf das Silber, in Form einer feinverteilten Phosphorverbindung mit einer Schmelz- bzw. Zersetzungstemperatur von mehr als 500°C (Phosphorverbindung P) in Kontakt bringt.

3. Verfahren nach Anspruch 1, dadurch gekennzeichnet, daß man ein phosphordotiertes Silberkatalysator-Festbett (b) einsetzt, welches erhältlich ist, indem man
Ib. Silberkristalle, die man durch elektrolytische Abscheidung von Silber aus einer wässerigen Silbersalzlösung erhält, zu einem Ausgangs-Silberkatalysator-Festbett (b) anordnet,
IIb.aus dem Ausgangs-Silberkatalysator-Festbett (b) ein aktiviertes Silberkatalysator-Festbett (b) herstellt, indem man durch dieses bei einer Temperatur von 150 bis 800°C eine Gasmischung, enthaltend Methanol und Sauerstoff, jedoch weniger als 0,1 Vol-% Distickstoffoxid (Gasmischung ii), oder die Gasmischung (i) leitet, und
IIIb.das aktivierte Silberkatalysator-Festbett (b) mit 1 bis 20000Gew.-ppm, Phosphor, bezogen auf das Silber, in Form einer feinverteilten Phosphorverbindung (P) in Kontakt bringt.

4. verfahren nach den Ansprüchen 1 bis 3, dadurch gekennzeichnet, daß man ein Ausgangs-Silberkatalysator-Festbett (a) oder (b), das aus einer oder mehreren Schichten von Silberkristallen, deren längster mittlerer Durchmesser 0,2 bis 10 mm beträgt, besteht, und wobei die Gesamtschichtdicke 1 bis 10 cm beträgt, einsetzt.

5. Verfahren nach den Ansprüchen 1 bis 3, dadurch gekennzeichnet, daß man pro cm² Querschnittsfläche des phosphordotierten Silberkatalysator-Festbetts und pro h 0,01 bis 1 kg Methanol in Form der der Gasmischung (i) durch dieses leitet.

6. Verfahren nach den Ansprüchen 1 bis 5, dadurch gekennzeichnet, daß man pro kg Methanol, das man in Form der Gasmischung (i) pro cm² der Querschnittsfläche des phosphordotierten Silberkatalysator-Festbetts (a) oder (b) durch dieses leitet, 0,01 bis 100Gew.-ppm Phosphor, bezogen auf das phosphordotierte Silberkatalysator-Festbett, in Form der feinverteilten Phosphorverbindung (P) auf das phosphordotierte Silberkatalysator-Festbett (a) oder (b) aufbringt , ohne die Durchleitung der Gasmischung (i) zu unterbrechen.

## Claims

1. A process for preparing formaldehyde by oxidative dehydrogenation of methanol, which comprises passing a gas mixture (i) containing
a) from 0.1 to 50% by volume of methanol,
b) from 0.1 to 30% by volume of oxygen,
c) from 0.1 to 50% by volume of dinitrogen oxide and
d) from 0 to 60% by volume of water
at from 150 to 800°C through a phosphorus-doped silver catalyst fixed bed.

2. A process as claimed in claim 1, wherein a phosphorus-doped silver catalyst fixed bed (a) is used which is obtainable by
Ia. arranging silver crystals obtained by electrolytic deposition of silver from an aqueous silver salt solution to form a starting silver catalyst fixed bed (a),
IIa.bringing the starting silver catalyst fixed bed into contact with from 1 to 20,000 ppm by weight of phosphorus, based on the silver, in the form of a finely divided phosphorus compound having a melting or decomposition temperature above 500°C (phosphorus compound P).

3. A process as claimed in claim 1, wherein a phosphorus-doped silver catalyst fixed bed (b) is used which is obtainable by
Ib. arranging silver crystals which are obtained by electrolytic deposition of silver from an aqueous silver salt solution to form a starting silver catalyst fixed bed (b),
IIb.preparing from the starting silver catalyst fixed bed (b) an activated silver catalyst fixed bed (b), by passing a gas mixture containing methanol and oxygen, but less than 0.1% by volume of dinitrogen oxide (gas mixture ii), or the gas mixture (i) through the silver catalyst fixed bed at from 150 to 800°C, and
IIIb.bringing the activated silver catalyst fixed bed (b) into contact with from 1 to 20,000 ppm by weight of phosphorus, based on the silver, in the form of a finely divided phosphorus compound (P).

4. A process as claimed in any of claims 1 to 3, wherein a starting silver catalyst fixed bed (a) or (b) is used which comprises one or more layers of silver crystals whose longest mean diameter is from 0.2 to 10 mm, and the total layer thickness being from 1 to 10 cm.

5. A process as claimed in any of claims 1 to 3, wherein from 0.01 to 1 kg of methanol is passed through the phosphorus-doped silver catalyst fixed bed in the form of the gas mixture (i) per cm² of its cross-sectional area and per h.

6. A process as claimed in any of claims 1 to 5, wherein from 0.01 to 100 ppm by weight of phosphorus, based on the phosphorus-doped silver catalyst fixed bed, in the form of the finely divided phosphorus compound (P) is applied to the phosphorus-doped silver catalyst fixed bed (a) or (b) per kg of methanol which is passed through the silver catalyst fixed bed (a) or (b) in the form of the gas mixture (i) per cm² of its cross-sectional area, without interrupting the throughflow of the gas mixture (i).

## Revendications

1. Procédé de préparation de formaldéhyde par déshydrogénation oxydante de méthanol, caractérisé en ce que l'on conduit un mélange de gaz (i), contenant
a) 0,1 à 50 % en volume de méthanol,
b) 0,1 à 30 % en volume d'oxygène,
c) 0,1 à 50 % en volume de dioxyde d'azote et
d) 0 à 60 % en volume d'eau,
à une température de 150 à 800°C à travers un lit fixe de catalyseur à l'argent dopé au phosphore.

2. Procédé selon la revendication 1, caractérisé en ce que l'on utilise un lit fixe de catalyseur à l'argent dopé au phosphore (a) pouvant être obtenu par
la. agencement de cristaux d'argent obtenus par séparation électrolytique d'argent à partir d'une solution aqueuse de sel d'argent, en un lit fixe de catalyseur à l'argent de départ (a) et
lla. mise en contact du lit fixe de catalyseur à l'argent de départ (a) avec 1 à 20000 ppm en poids de phosphore, par rapport à l'argent, sous forme d'un composé du phosphore finement divisé ayant une température de fusion ou de décomposition supérieure à 500°C (composé du phosphore P).

3. Procédé selon la revendication 1, caractérisé en ce que l'on utilise un lit fixe de catalyseur à l'argent dopé au phosphore (b) pouvant être obtenu par
lb. agencement de cristaux d'argent obtenus par séparation électrolytique d'argent à partir d'une solution aqueuse de sel d'argent, en un lit fixe de catalyseur à l'argent de départ (b),
llb. préparation d'un lit fixe de catalyseur à l'argent (b) activé à partir du lit fixe de catalyseur à l'argent de départ (b), où l'on conduit à travers lui un mélange de gaz contenant du méthanol et de l'oxygène mais moins de 0,1% en volume de dioxyde d'azote (mélange de gaz ii) ou le mélange de gaz (i), à une température de 150 à 800°C, et
lllb. mise en contact du lit fixe de catalyseur à l'argent (b) activé avec 1 à 20000 ppm en poids de phosphore, par rapport à l'argent, sous forme d'un composé du phosphore (P) finement divisé.

4. Procédé selon l'une quelconque des revendications 1 à 3, caractérisé en ce que l'on utilise un lit fixe de catalyseur à l'argent de départ (a) ou (b), construit à partir d'une ou plusieurs couches de cristaux d'argent dont le plus grand diamètre moyen est de 0,2 à 10 mm, et où l'épaisseur totale des couches vaut de 1 à 10 cm.

5. Procédé selon l'une quelconque des revendications 1 à 3, caractérisé en ce que l'on conduit, par cm² de la surface de la section du lit fixe de catalyseur à l'argent dopé au phosphore et par heure, 0,01 à 1 kg de méthanol sous forme du mélange de gaz (i) à travers celui-ci.

6. Procédé selon l'une quelconque des revendications 1 à 5, caractérisé en ce que l'on dépose par cm² de la surface de la section du lit fixe de catalyseur à l'argent dopé au phosphore (a) ou (b), et par kg de méthanol introduit sous forme du mélange de gaz (i) à travers celui-ci, 0,01 à 100 ppm en poids de phosphore, par rapport au lit fixe de catalyseur à l'argent dopé au phosphore, sous forme d'un composé du phosphore finement divisé (P), sur le lit fixe de catalyseur à l'argent dopé au phosphore (a) ou (b), sans interrompre le passage du mélange de gaz (i).
